Europäisches Patentamt

European Patent Office

Office européen des brevets

(11) Veröffentlichungsnummer : **0 346 270 B1**

(12) # EUROPÄISCHE PATENTSCHRIFT

(45) Veröffentlichungstag der Patentschrift :
**20.05.92 Patentblatt 92/21**

(51) Int. Cl.⁵ : **A61F 2/34**

(21) Anmeldenummer : **89730138.8**

(22) Anmeldetag : **01.06.89**

(54) Schraubpfanne als Teil einer Hüftgelenksprothese.

(30) Priorität : **26.08.88 DE 8810783 U**
**06.06.88 DE 8807481 U**

(43) Veröffentlichungstag der Anmeldung :
**13.12.89 Patentblatt 89/50**

(45) Bekanntmachung des Hinweises auf die
Patenterteilung :
**20.05.92 Patentblatt 92/21**

(84) Benannte Vertragsstaaten :
**AT BE CH DE ES FR GB GR IT LI NL SE**

(56) Entgegenhaltungen :
**EP-A- 0 065 482**
**DE-U- 8 810 783**
**FR-A- 2 597 329**
**MEDIZINISCH-ORTHOPAEDISCHE TECHNIK**

(73) Patentinhaber : **MECRON MEDIZINISCHE**
**PRODUKTE GMBH**
**Nunsdorfer Ring 23-29**
**W-1000 Berlin 48 (DE)**

(72) Erfinder : **Anapliotis, Emmanuel**
**Kiebitzweg 5**
**W-1000 Berlin 33 (DE)**
Erfinder : **Kranz, Curt, Dr.-Ing.**
**Kufsteiner Strasse 12**
**W-1000 Berlin 62 (DE)**
Erfinder : **Ploetz, Wiebke**
**Schmiljanstrasse 4**
**W-1000 Berlin 41 (DE)**

(74) Vertreter : **Christiansen, Henning, Dipl.-Ing.**
**Patentanwalt CHRISTIANSEN Pacelliallee**
**43/45**
**W-1000 Berlin 33 (DE)**

## Beschreibung

Die Erfindung betrifft eine Schraubpfanne der im Oberbegriff des Anspruchs 1 angegebenen Art.

Bei den herkömmlichen Schraubpfannen, wie sie beispielsweise aus der EP-A-0 065 482 bekannt sind, wird der Tragring dieser Schraubpfanne mit einem Schraubwerkzeug ohne Vorschneiden in die Acetabulumpfanne eingedreht und hat dort einen festen Sitz, der für eine frühzeitige Mobilität des Patienten ausreichend ist. In den Innenraum des Tragrings ist eine Innenpfanne aus Kunststoff einschnappbar, die als Lager für die Gelenkkugel dient. Die Kappe der Innenpfanne stützt sich durch die obere Ringöffnung des Tragrings hindurch im Acetabulum ab. Die Ringkonstruktion des Tragrings ermöglicht es, daß sich dieser durch entsprechende Verformung den lokalen Gegebenheiten im Acetabulum anpaßt, so daß der Gewindeeingriff und die Krafteinleitung physiologisch ohne lokale Überbeanspruchung der Knochenstruktur erfolgen kann. Auf diese Weise wird insbesondere ein Knochenabbau vermieden, wie er sonst im Bereich örtlicher Kraftspitzen vorkommt. Nachteilig bei dieser Schraubpfanne ist aber, daß ein Kunststoffabrieb an der Außenseite der eingeschnappten Innenpfanne erfolgen kann, wodurch die Einsatzdauer dieses stark belasteten Elements der Schraubpfanne verringert wird.

Bei einer weiteren aus der FR-A-2 597 329 bekannten Schraubpfanne ist der nicht vom Tragring umgebende Teil der Innenpfanne fast vollständig mit einer metallischen Abdeckkappe versehen, so daß die Schraubpfanne eine überwiegend geschlossene metallische Struktur aufweist. Nachteilig bei dieser Schraubpfanne ist aber, daß die Abdeckkappe direkt am Tragring anliegt, so daß die Steifigkeit der Schraubpfanne durch die metallische Abdeckkappe vergrößert wird. Zudem ist es bei einer elliptischen Verformung des Tragrings so, daß nur noch die Teilbereiche der Abdeckkappe an denen der verformte Tragring anliegt zur Krafteinleitung dienen. Eine derartige erhöhte Belastung von Teilbereichen kann die Einsatzdauer einer solchen Schraubpfanne erheblich verringern.

Der Erfindung liegt nun die Aufgabe zugrunde, eine Schraubpfanne der eingangs genannten Gattung anzugeben, welche derart ausgebildet ist, daß die Einleitung von im implantierten Zustand auftretenden Kräften selbst bei einer Verformung des Tragrings nicht zur zu starken Beanspruchung einzelner Teilbereiche der Schraubpfanne führt.

Diese Aufgabe wird mit den kennzeichnenden Merkmalen des Anspruchs 1 angegebenen gelöst.

Die Erfindung beruht auf der Erkenntnis, daß durch eine elastische Trennung der mit Gewindeflanken versehenen, in den Knochen eingreifenden Bereiche der Schraubpfanne von denjenigen, welche sich im Scheitel der Schraubpfanne an der Innenseite des Acetabulums abstützen, eine optimale Fixation möglich ist. Während sich die ringförmige Tragstruktur aus ihrer Kreisform heraus beim Einschrauben auch in eine angenähert elliptische Gestalt umformen kann, kann die Grundform der sphärischen Abdeckkappe erhalten bleiben. Die Schraubpfanne wird insbesondere durch die zusätzliche metallische Abdeckkappe nicht in unerwünschter Weise versteift. Zugleich wird durch einen ringförmigen Zwischenraum, der als Dehnungsfuge zwischen Abdeckkappe und Tragring dient, eine Umformung des Tragrings ermöglicht, die nicht zur starken Beanspruchung einzelner Teilbereiche der Abdeckkappe und des Tragrings führt.

Die Abstützfunktion, die der Kappenbereich der Schraubpfanne übernimmt, wird dadurch unterstützt, daß der Scheitelbereich "porocoated" ist, wobei durch die erzielte Porosität ein Anwachsen der Abdeckkappe an die Knochenoberfläche erfolgt, so daß die Festigkeit des Prothesensitzes weiter heraufgesetzt ist. Durch das Anwachsen der mit der Innenpfanne verbundenen Abdeckkappe verringern sich die im implantierten Zustand über den Tragring in den Knochen einzuleitenden Kräfte.

Der erfindungsgemäße Tragring weist weiterhin den Vorteil auf, daß die Tragflankenteile, welche nur durch eine schmale Ausnehmung (Schneidnut) voneinander getrennt sind, als Einheit tragend wirken, da die zwischen den Gewindeteilen verbleibende Spongiosa in der Lage ist, als "Brücke" zwischen diesen Tragflankenteilen zu fungieren und die entsprechenden Kräfte aufzunehmen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Ausführungsbeispiel des erfindungsgemäßen Tragrings in perspektiver Darstellung sowie

Figur 2 eine Schnittdarstellung des Ausführungsbeispiels gemäß Figur 1.

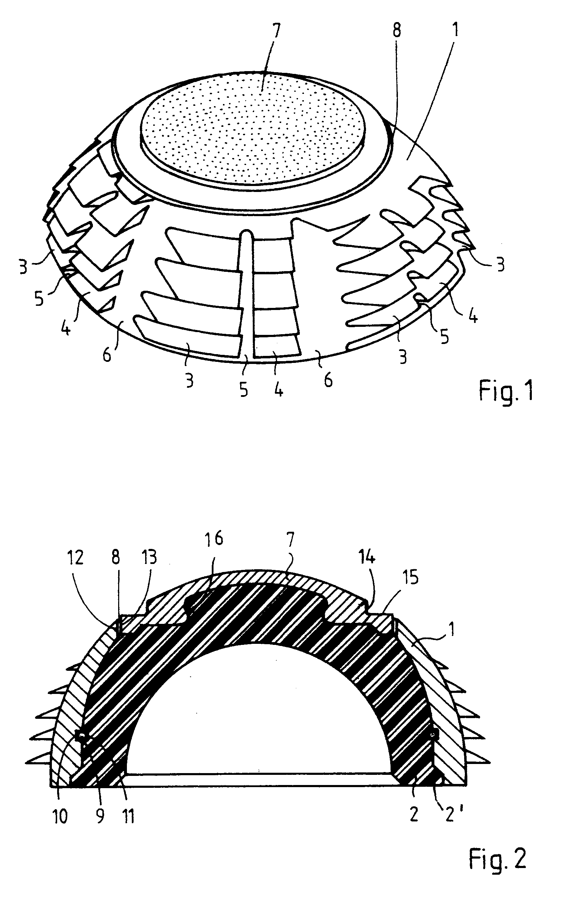

Der in Figur 1 dargestellte Tragring 1 ist aus Titan gefertigt und dient zur Aufnahme einer Kunststoff-Gelenkpfanne 2, die das Gelenklager bildet. Ein an der Innenwandung vorgesehener umlaufender, stufenförmiger Vorsprung 2' bildet ein Widerlager für den Halt der Pfanne.

Die Außenwandung des Tragrings ist sphärisch ausgebildet (bezogen auf den Gewindegrund), so daß der in unterschiedlichen Richtungen in ein ebenfalls sphärisch ausgefrästes Acetabulum einschraubbar ist, ohne daß weiterer Vorbereitungen, wie Gewindeschneiden, bedarf.

Tragflankengruppen 3 und 4 eines selbstschneidenden Gewindes sind von Schneidnuten 5 und 6 unterbrochen, welche einerseits die Späne beim selbstschneidenden Einschrauben aufnehmen und andererseits durch das Anwachsen der dort aufgenommenen Knochenspäne die Sekundärfixation der Pfanne ermöglichen.

Bei der Darstellung gemäß Fig. 1 ist die Kappe 7 auf den Tragring 1 aufgesetzt dargestellt. Es ist ersichtlich, daß sich zwischen Kappe und Tragring ein Spalt 8 von ca. 0,5 mm Breite befindet. Dieser bildet damit praktisch eine "Dehnungsfuge", welche den Ausgleich von Verformungen des Tragrings 1 beim Einschrauben in das Acetabulum ausgleicht.

Die nach außen weisende Oberfläche der Kappe ist mit einer porösen Schicht (porocoat) versehen, wobei die Schicht aus Reintitan aufgesintert ist und zwar in einem isostatischen Heißverfahren unter hohem Druck. Die so gebildete poröse Oberflächenschicht begünstigt das Anwachsen der äußeren Fläche der Kappe 7 im angrenzenden Knochenbereich und unterstützt somit die Sekundärfixation.

In Fig. 2 ist die zusammengesetzte Pfanne im Schnitt dargestellt. In den Tragring 1, der ebenfalls aus Titan besteht, ist eine Polyethylen-Innenpfanne eingesetzt, wobei die gegenseitige Befestigung durch einen Metallring 9 vorgenommen wird, der in Nuten 10 und 11 des Tragrings bzw. der Innenpfanne verläuft. Diese Verbindung ist bekannt und entspricht der herkömmlichen Ausführung einer aus einem äußeren Metalltragring und einer Polyethylen-Innenpfanne bestehenden Hüftpfanne.

Im Scheitel der Innenpfanne 2 ist der Querschnitt der Kappe 7 ausgespart, wobei sich die Außenoberflächen des Rings 1 und der Kappe 7 zu einer sphärischen Kontur ergänzen.

Auf diese Weise ist sichergestellt, daß die zusammengesetzte Pfanne in ein halbkugelförmig ausgefrästes Acetabulum in unterschiedlichen Raumrichtungen eingeschraubt werden kann, ohne daß ein ausgerichtetes Fräsen und Gewindevorschneiden erforderlich ist.

Die Innenpfanne und die Kappe 7 weisen eine Schnappverbindung auf, die derjenigen eines Druckknopfs entspricht. Dabei ist die Innenpfanne mit einem konkaven Ansatz 16 versehen, der in eine entsprechende konkave Aussparung eingreift. Das Fixieren nach dem Einschnappen erfolgt dabei - entsprechend dem Druckknopf-Prinzip - durch Hinterschneidungen.

Die obere Öffnung des Traggings 1 und die Kappe 7 grenzen im Bereich des Spaltes 8 mit einem Abstand von ca. 0,5 mm im Bereich zweier zylindrischer Flächen 12 bzw. 13 aneinander, welche Zylindermantelflächen bilden und konzentrisch ausgerichtet sind. Auf diese Weise ist für einen Toleranzausgleich zwischen Tragging 1 und Kappe 7 auch bei der Einleitung von im implantierten Zustand auftretenden Kräften gesorgt.

Über ein ringförmige Stufe 14 grenzt ein ebenfalls ringförmiger Randbereich 15 der Kappe 7 an den mit einer Porocoatschicht versehenen zentralen Bereich. Damit beschränkt sich der letztgenannte Bereich auf denjenigen Teil der Pfanne, bei dem die im wesentlichen parallel zur Mittelachse gerichtete Krafteinleitung stattfindet. Die durch die Stufe 14 gebildete Kante gibt der Kappe im implantierten Zustand selbständig Halt am angrenzenden Knochenbereich, so daß insoweit die Verbindung zwischen Pfanneneinsatz 2 und Kappe 7 nur gering belastet ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Schraubpfanne als Teil einer Hüftgelenksprothese, bestehend aus einem äußeren Tragring (1), einer in den Tragring (1) einsetzbaren Kunststoff-Innenpfanne (2) und einer mindestens eine metallische Oberfläche aufweisenden Abdeckkappe (7),
**dadurch gekennzeichnet ,**
daß die Abdeckkappe (7) den nicht vom Tragring (1) umgebenen Teil der Innenpfanne (2) abdeckt und dessen metallene Außenkontur zu der sphärischen Form einer im wesentlichen geschlossenen Halbkugel oder Kugelkappe ergänzt, und daß zwischen Abdeckkappe (7) und Tragring (1) bei zusammengesetzter Schraubpfanne ein ringförmiger Zwischenraum (8) besteht.

2. Schraubpfanne nach Anspruch 1, **dadurch gekennzeichnet ,** daß die Abdeckkappe (7) eine zylindrische Außenwandung (13) aufweist, die bei zusammengesetzter Schraubpfanne parallel zu einer entsprechenden Innenwandung (12) des Tragrings (1) verläuft.

3. Schraubpfanne nach Anspruch 2, **dadurch gekennzeichnet,** daß die Abdeckkappe (7) ausschließlich mit der Innenpfanne (2) mechanisch verbunden ist.

4. Schraubpfanne nach Anspruch 3, **dadurch gekennzeichnet,** daß die Abdeckkappe (7) auf die Innenpfanne (2) aufschnappbar ist.

5. Schraubpfanne nach Anspruch 4, **dadurch gekennzeichnet,** daß die Aufschnappvorrichtung (16) der Form eines Druckknopfes entspricht.

3

6. Schraubpfanne nach einem der vorangehenden Ansprüche, dadurch **gekennzeichnet**, daß die Abdeckkappe (7) aus Titan besteht.

7. Schraubpfanne nach einem der vorangehenden Ansprüche, dadurch **gekennzeichnet**, daß die Außenfläche der Abdeckkappe (7) porocoated ist.

8. Schraubpfanne nach Anspruch 7, **dadurch gekennzeichnet**, daß ein Randbereich (15) der Außenfläche der Abdeckkappe (7) nicht porocoated ist.

9. Schraubpfanne nach Anspruch 8, **dadurch gekennzeichnet**, daß der Randbereich (15) mit einer konzentrischen Stufe (14) versehen ist, die einen zylindrischen, nach außen gerichteten, blanken Wandungsbereich aufweist.

## Claims

1. Screw cup as part of a hip joint prosthesis, consisting of an outer support ring (1), a plastic inner cup (2) which can be fitted into the support ring (1), and a cover cap (7) having at least a metallic upper surface, characterised in that
the cover cap (7) covers that part of the inner cup (2) not surrounded by the support ring (1) and completes the metal outer contour thereof to give the spherical shape of an essentially closed hemisphere or spherical cap, and in that, with the screw cup assembled, there is an annular intermediate space (8) between cover cap (7) and support ring (1).

2. Screw cup according to Claim 1, characterised in that the cover cap (7) has a cylindrical outer wall (13) which, with the screw cup assembled, extends in parallel with a corresponding inner wall (12) of the support ring (1).

3. Screw cup according to Claim 2, characterised in that the cover cap (7) is mechanically connected exclusively to the inner cup (2).

4. Screw cup according to Claim 3, characterised in that the cover cap (7) can be snapped onto the inner cup (2).

5. Screw cup according to Claim 4, characterised in that the snap-on arrangement (16) corresponds to the shape of a snap-button.

6. Screw cup according to one of the preceding claims, characterised in that the cover cap (7) consists of titanium.

7. Screw cup according to one of the preceding claims, characterised in that the outer surface of the cover cap (7) is porocoated.

8. Screw cup according to Claim 7, characterised in that an edge area (15) of the outer surface of the cover cap (7) is not porocoated.

9. Screw cup according to Claim 8, characterised in that the edge area (15) is provided with a concentric step (14), which has a cylindrical, outwardly directed, blank wall area.

## Revendications

1. Corps de cavité à pas de vis faisant partie d'une prothèse de cavité cotyloïde, se composant d'un anneau extérieur de support (1), d'une cuvette intérieure de matière plastique (2) se logeant dans l'anneau de support (1) et d'un capuchon de couverture (7) comportant au moins une surface métallique, caractérisé en ce que
le capuchon de couverture (7) recouvre la partie de la cuvette intérieure (2) qui n'est pas enveloppée par l'anneau de support (1) et son contour extérieur métallique forme le complément de la forme sphérique d'une hémisphère ou d'un capuchon sphérique sensiblement fermé et en ce qu'il subsite un interstice annulaire (8) entre le capuchon de couverture (7) et l'anneau de support (1) lorsque le corps de cavité à pas de vis est assemblé.

2. Corps de cavité à pas de vis selon la revendication 1, caractérisé en ce que le capuchon de couverture (7) comporte une paroi extérieure cylindrique (13) qui, après assemblage du corps de cavité à pas de vis, est parallèle à une paroi intérieure correspondante (12) de l'anneau de support (1).

3. Corps de cavité à pas de vis selon la revendication 2, caractérisé en ce que le capuchon de couverture (7) est relié mécaniquement exclusivement à la cuvette intérieure (2).

4. Corps de cavité à pas de vis selon la revendication 3, caractérisé en ce que le capuchon de couverture (7) s'enclenche élastiquement sur la cuvette intérieure (2).

5. Corps de cavité à pas de vis selon la revendication 4, caractérisé en ce que le dispositif d'enclenchement élastique (16) correspond à la forme d'un bouton pression.

6. Corps de cavité à pas de vis selon l'une des revendication précédentes, caractérisé en ce que le capuchon de couverture (7) est en titane.

7. Corps de cavité à pas de vis selon l'une des revendications précédentes, caractérisé en ce que la surface extérieure du capuchon de couverture (7) comporte un revêtement poreux.

8. Corps de cavité à pas de vis selon la revendication 7, caractérisé en ce qu'une zone de bordure (15) de la surface extérieure du capuchon de couverture (7) ne comporte aucun revêtement poreux.

9. Corps de cavité à pas de vis selon l'une des revendications précédentes, caractérisé en ce la zone de bordure (15) comporte un étagement concentrique (14) qui présente une zone cylindrique de paroi nue orientée vers l'extérieur.

Fig. 1

Fig. 2